# EUROPEAN PATENT SPECIFICATION

(11) **EP 4 547 318 B1**
(45) Date of publication and mention of the grant of the patent: **11.03.2026**
(21) Application number: 23738138.9
(22) Date of filing: 12.06.2023
(51) Int. Cl.: A61N 1/36, A61N 1/362, A61N 1/365, A61B 5/0538, A61B 5/00, A61B 5/257, A61B 5/06

(54) **TEMPORARY PACING LEAD NAVIGATION SYSTEM**
NAVIGATIONSSYSTEM MIT TEMPORÄRER SCHRITTMACHERLEITUNG
SYSTÈME DE NAVIGATION DE SONDE DE STIMULATION CARDIAQUE TEMPORAIRE

(30) Priority: 01.07.2022 US 202263367548 P
(43) Date of publication of application: 07.05.2025
(73) Proprietor: Medtronic, Inc., Minneapolis, Minnesota 55432 (US)
(72) Inventor: MARSHALL, Mark T., Minneapolis, Minnesota 55432 (US); WHITMAN, Teresa A., Minneapolis, Minnesota 55432 (US)
(74) Representative: Zimmermann & Partner Patentanwälte mbB
(86) International application number: PCT/IB2023/056050
(87) International publication number: WO 2024/003644

(56) References cited:
- WO-A1-2020/247619
- US-A1- 2009 264 739
- US-A1- 2021 268 297
- US-A1- 2022 117 656

## Description

### TECHNICAL FIELD

This disclosure relates to medical device systems and, more particularly, to medical device systems for delivery of electrical stimulation therapy.

### BACKGROUND

In some situations, medical professionals may insert an implantable medical lead into the body of the patient to deliver various therapies. In some examples, the implantable medical leads may be temporary and may be removed from the patient after a period of time. In some examples, with respect to cardiac-related issues, a medical professional may insert a type of implantable medical lead also referred to as a temporary pacing lead into a heart of the patient and deliver temporary cardiac pacing to the heart.

### SUMMARY

The invention is defined in independent claims 1 and 9, further embodiments are described in respective dependent claims 2-8 and 10-15. The devices, systems, and techniques of this disclosure generally relate to navigating an implantable medical lead through vasculature of a patient to a target treatment site. In some situations, a medical professional may need to implant the implantable medical lead without access to standard imaging devices and systems (e.g., fluoroscopy). This disclosure describes devices, systems, and techniques for determining the position of the implantable medical lead within the body of the patient using surface electrodes attached to the patient.

In an example, the disclosure describes a non-claimed method, useful for the understanding of the invention, method including outputting, by a computing system through a lead electrode on a distal portion of an implantable medical lead within vasculature of a patient, an electrical signal, wherein the implantable medical lead further comprises an expandable member that is in an expanded state while the electrical signal is outputted, wherein the expandable member is configured, when expanded to directionally-impede the electrical signal; sensing, by the computing system and through each of a plurality of surface electrodes positioned on the patient, the electrical signal; determining, by the computing system for each respective surface electrode of the plurality of surface electrodes, a value of the sensed electrical signal corresponding to an impedance between the respective surface electrode and the lead electrode; and determining, by the computing system, a position and an orientation of the distal portion of the implantable medical lead within the vasculature of the patient based on the determined values of the sensed electrical signal.

**In** some examples, the disclosure describes a computer system including a memory; sensing circuitry coupled to a plurality of surface electrodes placed onto skin of the patient; signal generation circuitry coupled to a lead electrode on a distal portion of an implantable medical lead within vasculature of a patient; and processing circuitry coupled to the memory, the sensing circuitry and the signal generation circuitry, the processing circuitry configured to: output an electrical signal from the lead electrode within vasculature of a patient, wherein the electrical signal is directionally impeded by an expandable member of the implantable medical lead in an expanded configuration; sense the electrical signal through each of the plurality of surface electrodes; determine, for each respective surface electrode of the plurality of surface electrodes, a value of the sensed electrical signal corresponding to an impedance between the respective surface electrode and the lead electrode; and determine a position and an orientation of the distal portion of the implantable medical lead within the vasculature of the patient based on the determined values of the sensed electrical signal.

In some examples, the disclosure describes a computer readable storage medium including instructions that, when executed, cause processing circuitry within a device to: output, through a lead electrode on a distal portion of an implantable medical lead within vasculature of a patient, an electrical signal, wherein the implantable medical lead further comprises an expandable member that is in an expanded state while the electrical signal is outputted, wherein the expandable member is configured, when expanded to directionally-impede the electrical signal; sense, through each of a plurality of surface electrodes positioned on the patient, the electrical signal; determine, for each respective surface electrode of the plurality of surface electrodes, a value of the sensed electrical signal corresponding to an impedance between the respective surface electrode and the lead electrode; and determine a position and an orientation of the distal portion of the implantable medical lead within the vasculature of the patient based on the determined values of the sensed electrical signal.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a conceptual diagram of a medical device system for navigating an implantable medical lead within a patient.
FIG. 2 is a block diagram illustrating an example configuration of a medical device of the system of FIG. 1.
FIG. 3 is a conceptual diagram illustrating an example process of determining the location of an implantable medical lead within the patient.
FIG. 4 is a conceptual diagram illustrating a distal portion of the implantable medical lead in a retracted configuration.
FIG. 5 is a conceptual diagram illustrating a distal portion of the implantable medical lead in an extended configuration.
FIG. 6 is a conceptual diagram illustrating a distal portion of the implantable medical lead of FIG. 5 with an expandable member in an expanded configuration.
FIG. 7 is a conceptual diagram illustrating a cross-sectional view of distal portion of the implantable medical lead of FIG. 6 along line A-A.
FIG. 8 is a flowchart illustrating an example process of navigating an implantable medical lead within body of a patient.

### DETAILED DESCRIPTION

Medical devices, systems, and techniques of this disclosure relates to the navigation of an implantable medical lead through vasculature of a patient to a target treatment site. A medical professional may insert an implantable medical lead into the patient (e.g., through vasculature of the patient) to deliver an implantable medical device, deliver therapies and/or treatments, retrieve samples, and the like. During the insertion process, the medical professional needs to keep track of the position of the implantable medical lead within the patient. In some situations, such as outside of a clinical environment, a medical professional may need to insert an implantable medical lead into a patient while the medical professional does not have access to an standard imaging devices and processes such as ultrasound, fluoroscopy, x-ray imaging, computed tomography (CT), magnetic resonance imaging (MRI), optical coherence tomography (OCT), or the like.

This disclosure provides improvements over other implantable medical lead systems and insertion processes by allowing a medical professional to insert the implantable medical lead without requiring standard imaging devices and processes which may be difficult to access in certain environments. The medical devices, systems, and techniques of this disclosure also provides a simplified implantable medical lead navigation process which may reduce set-up time, complexity, and costs. The medical devices, systems, and techniques of this disclosure may also enable identification of an orientation of the distal end of the implantable medical lead in addition to its position.

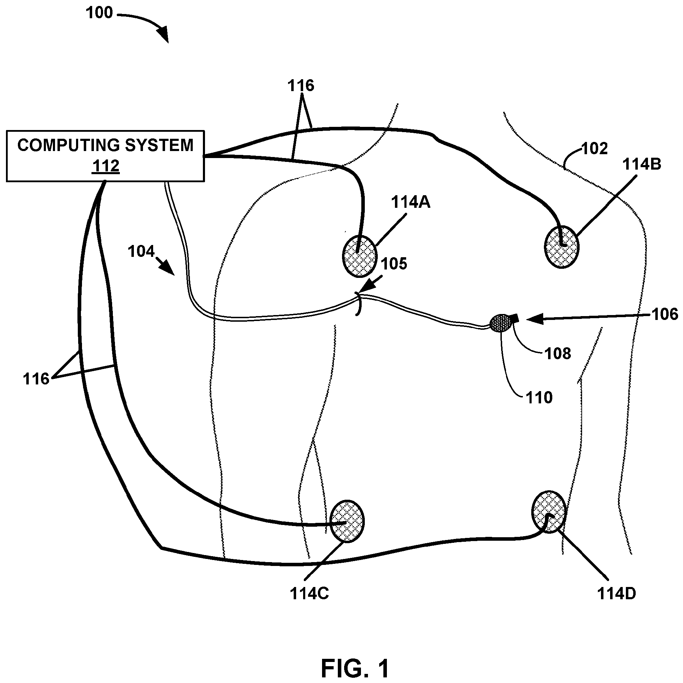

FIG. 1 is a conceptual diagram of a medical device system 100 for navigating an implantable medical lead 104 within a patient 102. Medical device system 100 may include computing system 112, implantable medical lead 104, and surface electrodes 114A-114D (hereinafter referred to as "surface electrodes 114"). While FIG. 1 illustrates medical device system 100 having one computing system 112, in other examples the functions of computing system 112 may be performed by one or more computing and/or other devices, one or more computing systems, or a cloud computing environment.

Implantable medical lead 104 may be connected to computing system 112, e.g., to an external pacemaker or pacing system analyzer of computing system 112. A medical professional may insert implantable medical lead 104 into patient 102 at an insertion site 105. While the insertion site 105 of FIG. 1 is illustrated to be in the upper thorax or clavicular area of patient 102, the insertion site 105 may be at other locations on patient 102 (e.g., the groin, the neck, the abdomen, and the like). The medical professional may insert distal portion 106 of implantable medical lead 104 into vasculature of patient 102 at insertion site 105 and navigate implantable medical lead 104 to a target location within patient 102 through vasculature of patient 102.

Distal portion 106 of implantable medical lead 104 may include a lead electrode 108 and an expandable member 110. In some examples, as illustrated in FIG. 1, lead electrode 108 may be positioned on a distal tip of implantable medical lead 104. Lead electrode 108 may be configured to transmit an electrical signal. Computing system 112 may be electrically connected to lead electrode 108 via one or more conductors disposed within implantable medical lead 104 and may deliver the electrical signal to lead electrode 108 for transmission.

Expandable member 110 may be positioned proximal to lead electrode 108. Expandable member 110 may be configured to transition from a collapsed state to an expanded state and vice versa. In some examples, expandable member 110 may be a balloon (e.g., a compliant balloon). The medical professional may expand or collapse expandable member 110 by delivering a fluid into an inner volume of expandable member 110 or remove the fluid from the inner volume of expandable member 110, respectively. In some examples, expandable member 110 may expand around and obscure portions of lead electrode 108. While expandable member 110 is expanded around lead electrode 108, expandable member 110 may block or at least partially obscure or impede the electrical signal transmitted by lead electrode 108. Expandable member 110 may be formed from one or more expandable materials. The expandable materials may include, but are not limited to, latex rubber, silicone rubber, or thermoplastic elastomers (e.g., polyurethane, ChronoPrene^{®}, or the like).

The medical professional may expand expandable member 110 once distal portion 106 of implantable medical lead 104 enters the heart of patient 102. In some examples, expandable member 110 may protect tissue of the heart from distal portion 106, e.g., lead electrode 108 on distal portion 106. In some examples, expandable member 110 may be propelled by blood flow within the heart and may facilitate further advancement of distal portion 106 of implantable medical lead 104 within the heart.

In some examples, expandable member 110 may include a plurality of sensors positioned on an external surface of a lead body of implantable medical lead 104. The plurality of sensors may be electrically connected to computing system 112 or another computing device and/or system of medical system 100 through implantable medical lead 104. Once expandable member 110 is in an expanded configuration within the heart, computing system 112 may detect pressure within expandable member 110 on the lead body through the plurality of sensors. Different detected pressures may correspond to different levels of obscuration of lead electrode 108 by expandable member 110. The different levels of obscuration may affect the magnitude and/or directional vector of the electrical path of the signal and thus the electrical impedance between lead electrode 108 and one or more surface electrodes 114. Expandable member 110 may exert pressure on the lead body in response to pressures exerted on expandable member 110 by the blood flow of the heart. In some examples, the plurality of sensors may transmit the detected pressure to computing system 112, which may then determine an orientation of distal portion 106 within the heart based at least in part on the detected pressure. In some examples, the detected pressure may be pulsatile and may relate to the cardiac cycle of the heart.

Medical system 100 may also include surface electrodes 114 disposed on patient 102. Surface electrodes 114 may be electrically connected to computing system 112 via electrical connectors 116. In some examples surface electrodes 114 may be electrically connected to another computing device and/or system of medical system 100 that is separate from computing system 112. Each of surface electrodes 114 is configured to sense the electrical signal transmitted by lead electrode 108 through tissue of patient 102. Each of surface electrodes 114 may be further configured to transmit the sensed electrical signal to computing system 112 or another computing device and/or system of medical system 100.

Each of surface electrodes 114 may be attached to skin of patient 102 (e.g., via patches, as illustrated in FIG. 1). In some examples, as illustrated in FIG. 1, medical system 100 may include four surface electrodes 114 (e.g., surface electrodes 114A, 114B, 114C, 114D) attached to the torso of patient 102 at the pectoral and hip regions. In other examples, medical system 100 may include fewer, e.g., three, surface electrodes 114 or five or more surface electrodes 114 and may be placed at other regions of patient 102 (e.g., on the upper back of patient 102, lower back of patient 102, limbs of patient 102, or the like).

Computing system 112 may determine, for each of surface electrodes 114, a value of the sensed electrical signal corresponding to an impedance between the respective surface electrode 114 and lead electrode 108. The impedance between the respective surface electrode 114 and lead electrode 108 may reflect the electrical opposition of the tissue of patient 102 to the electrical signal between lead electrode 108 and the respective surface electrode 114. The electrical opposition of the tissue of patient 102 corresponds to the distance between lead electrode 108 and the respective surface electrode 114. For example, computing system 112 may determine that surface electrodes 114 that are closer to lead electrode 108 (e.g., surface electrode 114B) have values of the sensed electrical signal corresponding to a relatively lower impedance value than surface electrodes 114 that are farther from lead electrode 108 (e.g., surface electrode 114C).

Computing system 112 may use the values of the sensed electrical signals to calculate linear distances between the lead electrode 108 and each of surface electrodes 114. Computing system 112 may determine the position and/or orientation of lead electrode 108 and implantable medical lead 104 within the vasculature of patient 102 through triangulation based on the positions of surface electrodes 114 on patient 102 and the respective linear distance for each of surface electrodes 114.

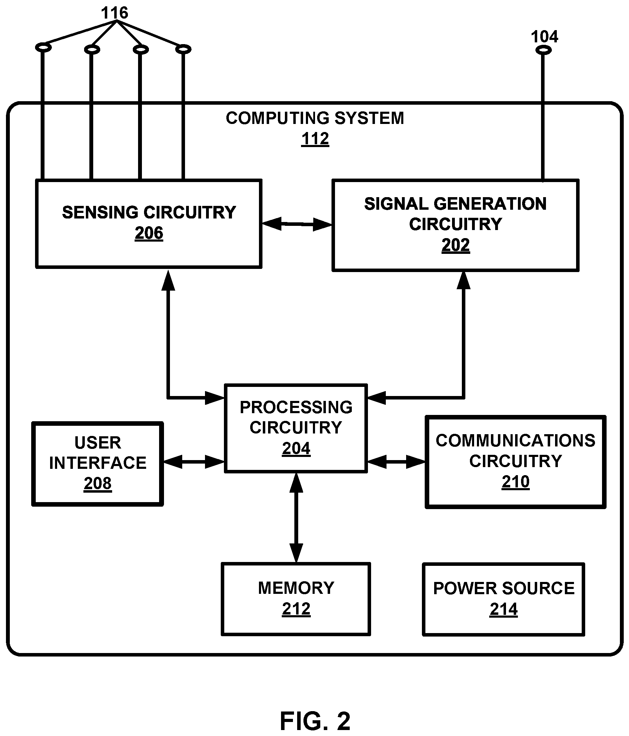

FIG. 2 is a block diagram illustrating an example configuration of a computing system 112 of the medical device system 100 of FIG. 1. Computing system 112 may include signal generation circuitry 202, processing circuitry 204, sensing circuitry 206, user interface (UI) 208, communications circuitry 210, memory 212, and power source 214. In other examples, computing system 112 may have additional components. In some examples, as illustrated in FIG. 2, the components of computing system 112 may be contained within a single computing device. In other examples, the components of computing system 112 may be contained in multiple computing devices, computing systems, other devices, and/or a cloud computing environment.

The various circuitry may be, or include, programmable or fixed function circuitry configured to perform the functions attributed to respective circuitry. Memory 212 may store computer-readable instructions that, when executed by processing circuitry 204, cause computing system 112 and/or implantable medical lead 104 to perform various functions. Memory 212 may be a storage device or other non-transitory medium.

Signal generation circuitry 202 is configured to generate electrical signals and may include, as examples, current or voltage sources, modulation circuitry or other signal generation circuitry. Signal generation circuitry 202 may be coupled to implantable medical lead 104. Signal generation circuitry 202 may be electrically connected to lead electrode 108 on distal portion 106 of implantable medical lead 104, e.g., to transmit the electrical signal to surface electrodes 114. In some examples, lead electrode 108 may act as an anode and one or more surface electrodes 108 may act as a cathode. The electrical signal may be a constant signal or may be a plurality of bursts of electrical signal. In some examples, signal generation circuitry 202 may also be configured to transmit electrical signals through lead electrode 108 to tissue of patient 102 for purposes of stimulation, therapy, treatment, or the like.

Sensing circuitry 206 may be connected to surface electrodes 114 via electrical connectors 116. Sensing circuitry 206 may record sensed electrical signals from one or more of surface electrodes 114 by receiving electrical signals delivered from the one or more surface electrodes 114 through the corresponding electrical connectors 116. Sensing circuitry 206 may be coupled to processing circuitry 204 and may provide the sensed electrical signals to processing circuitry 204. Processing circuitry 204 may determine, for each of the transmitted sensed electrical signals, the identification of the corresponding surface electrode (e.g., surface electrodes 114A, 114B, 114C, 114D, or the like). In some examples, the sensed electrical signals may represent the voltage or the current of the electrical signal sensed by surface electrodes 114.

Processing circuitry 204 may include any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 204. The functions attributed to processing circuitry 204 herein may be embodied as firmware, hardware, software, or any combination thereof.

Processing circuitry 204 may be configured to determine, for each of the sensed electrical signals transmitted from sensing circuitry 206, a value corresponding to the impedance between lead electrode 108 and the surface electrode 114 corresponding to the sensed electrical signal.

In some examples, the value may include amplitude, voltage, the impedance value, or the like. The impedance between lead electrode 108 and each of surface electrodes 114 may vary based on the distance. For example, if the electrical signal travels for a longer distance with tissue of patient 102, the impedance to the travel of the electrical current may be greater and the corresponding surface electrode 114 may sense an electrical signal with a reduced amplitude and/or voltage. Processing circuitry 204 may determine the distance between lead electrode 108 and each of surface electrodes 114 using the changes in amplitude and/or voltage between the two electrodes and a rate of increase of impedance and/or resistance in tissue of patient 102.

In some examples, processing circuitry 204 may determine the distance based on the specific anatomy of patient 102, such as the body fat percentage of patient 102, e.g., by performing bioelectrical impedance analysis (BIA) on patient 102. In some examples, processing circuitry 204 may determine the distance based on data from a sample population with similar traits to patient 102. The traits may include, but are not limited to, height, weight, body fat percentage, gender, and the like. In some examples, processing circuitry 204 may receive data from patient 102, a medical professional, and/or another individual via UI 208 and/or communications circuitry 210.

Based on the determined values corresponding to the impedance between lead electrode 108 and surface electrodes 114, processing circuitry 204 may determine the position of lead electrode 108 in patient 102 using triangulation. A medical professional may enter the positions of each of surface electrodes 114 on body of patient 102 into processing circuitry 204 and/or may place surface electrodes 114 at predetermined locations known to processing circuitry 204. For each of surface electrodes 114, processing circuitry 204 may determine a linear distance between lead electrode 108 and the respective surface electrode 114 based on the sensed value. For example, processing circuitry 204 may determine the linear distance based on a magnitude of the sensed value corresponding to impedance, e.g., in relation to a magnitude of the signal delivered by signal generation circuitry 202. In some examples, processing circuitry 204 may use the amplitude, the voltage, or another trait of the sensed electrical signals in the determined values to determine the linear distance.

For each of surface electrodes 114, processing circuitry 204 may generate a three-dimensional (3D) sphere round the respective surface electrode 114 indicating the range of potential locations of lead electrode 108. The 3D sphere has a diameter of the determined linear distance for the respective surface electrode 114. Using the 3D spheres from a plurality of surface electrodes 114, processing circuitry 204 may identify a single point within patient 102 where the 3D spheres intersect and may determine that lead electrode 108 is positioned at the identified point.

In some examples, processing circuitry 204 may then determine the orientation of lead electrode 108 based on the sensed electrical signals of surface electrodes 114. In some examples expandable member 110, when expanded around a portion of lead electrode 108, may block and/or reduce the electrical signal transmitted to surface electrodes 114. The orientation of expandable member 110 may affect the directional vectors of the electrical impedance. In some examples, the direction of obscuration of the expandable member 110 on lead electrode 108 may alter the directional vectors of the current between lead electrode 108 and one or more surface electrodes 114, and thus the electrical impedance. Processing circuitry 204 may determine the orientation of a portion of lead electrode 108 not obscured by expandable member 110 based on the directional vectors of the electrical signals sensed by surface electrodes 114.

In some examples, processing circuitry 206 may be configured to send to signal generation circuitry 202 instructions to transmit electrical signals through lead electrode 108 to surface electrodes 114. Processing circuitry 206 may send the instructions and determine the position and/or orientation of implantable medical lead 104 constantly, after passage of a period of time (e.g., a number of second, minutes, or the like), or in response to input from a medical professional (e.g., via UI 208 and/or communications circuitry 210). In some examples, processing circuitry 206 may send to signal generation circuitry 202 and switch circuitry instructions to transmit electrical signals to tissue of patient 102 for purposes of medical treatment, therapy, stimulation, or the like.

UI 208 may transmit information and/or retrieve data and instructions from a user, such as a medical professional. In other examples, UI 208 may display the position and/or orientation of implantable medical lead 104 (e.g., of distal portion 106 of implantable medical lead 104). In some examples, UI 208 may display a 3D representation of patient 102 and indication the position and/or orientation of implantable medical lead 104 within the 3D representation. In some examples, UI 208 may display a 3D representation of vasculature of patient 102. A user may use UI 208 of computing system 112 to navigate implantable medical lead 104 within vasculature of patient 102.

Communications circuitry 210 supports wireless and/or wired communication between computing system 112 and one or more external computing devices and/or systems. In some examples, communications circuitry 210 may support communications between multiple computing devices under the control of processing circuitry 204. Communications circuitry 210 may accomplish communication by radiofrequency (RF) communication techniques, e.g., via an antenna, wired communication techniques, or any other communication techniques.

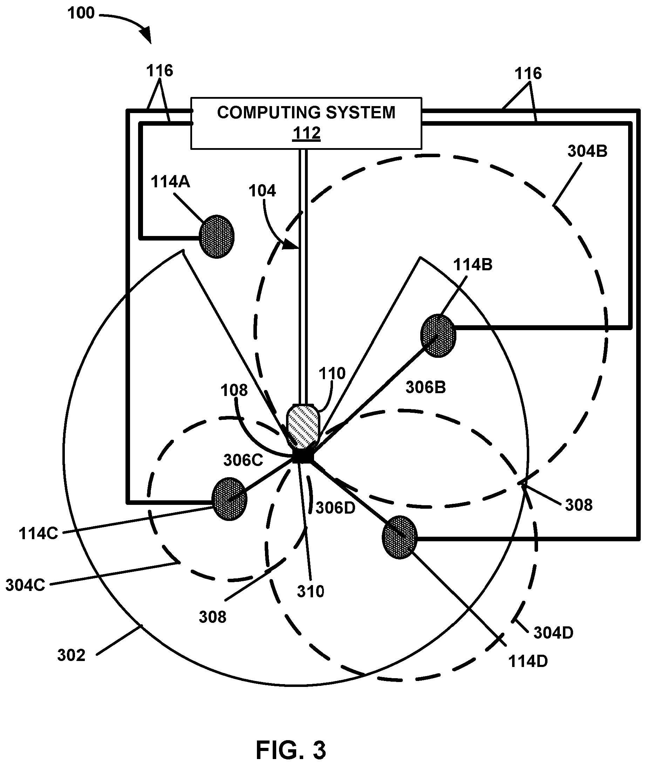

FIG. 3 is a conceptual diagram illustrating an example process of determining the location of implantable medical lead 104 within the patient. As illustrated in FIG. 3, medical device system 100 may include computing system 112, implantable medical lead 104, and surface electrodes 114. Implantable medical lead may be electrically coupled to computing system 112 and may include lead electrode 108 and expandable member 110, shown in the expanded configuration in FIG. 3. Surface electrodes 114 are coupled to computing system 112 via electrical connectors 116. While the example process illustrated in FIG. 3 includes four surface electrodes 114A-114D, other example processes may have three surface electrodes 114 or four or more surface electrodes 114.

Computing system 112 may instruct lead electrode 108 to transmit electrical signal 302 through body of patient 102 (not shown in FIG. 3). Lead electrode 108 may transmit electrical signal which results in field in the shape of a sphere, a partial sphere, a toroid, or another shape around body of lead electrode 108. The shape of the filed may be a result of the designs of lead electrode 108 and expandable member 110, and their orientation relative to one another.

One or more of surface electrodes 114 (e.g., surface electrodes 114B, 114C, and 114D) may sense electrical signal 108 and transmit the sensed electrical signal to computing system 112. Depending on the impedance of tissue of patient 102 and distance of each of the one or more of surface electrodes 114 from lead electrode 108, the one or more of surface electrodes 114 may sense electrical signal 302 at different magnitudes. For examples, since surface electrode 114C is closer to lead electrode 108 than surface electrode 114B, surface electrode 114C may sense electrical signal 302 having a higher magnitude than surface electrode 114B.

Based on the sensed electrical signals, computing system 112 may determine linear distances 306B-306D (hereinafter referred to as "linear distances 306) for surface electrodes 114 and generate spheres 304B-304D (hereinafter referred to as "spheres 304) around each of surface electrodes 114. Spheres 304 may represent possible positions of lead electrode 108 relative to the corresponding surface electrodes 114. Spheres 304 have diameters corresponding to the determined linear distances 306 for the corresponding surface electrodes 114. Using spheres 304, computing system 112 may determine points of intersection 308 between two or more of spheres 304. Computing system 112 may determine, from points of intersection 308, a point 310 where all of spheres 304 intersect and determine that the position of lead electrode 108 is at point 310.

In some examples, one or more of surface electrodes 114 (e.g., surface electrode 114A), may not sense electrical signal 302 or may sense an impeded electrical signal from lead electrode 108. Electrical signal 302 may be blocked or directionally-impeded by expandable member 110. Computing system 112 may determine that surface electrode 114A does not sense electrical signal 302 (or that the magnitude of signal 302 is below a threshold indicative that surface electrode 114A is obscured from electrode 108 by expandable member 110) and may determine an orientation of lead electrode 108 where lead electrode 108 is directionally blocked or impeded by expandable member 110 from transmitting electrical signal 302 to surface electrode 114A but not to other surface electrodes 114 (e.g., surface electrodes 114B-D).

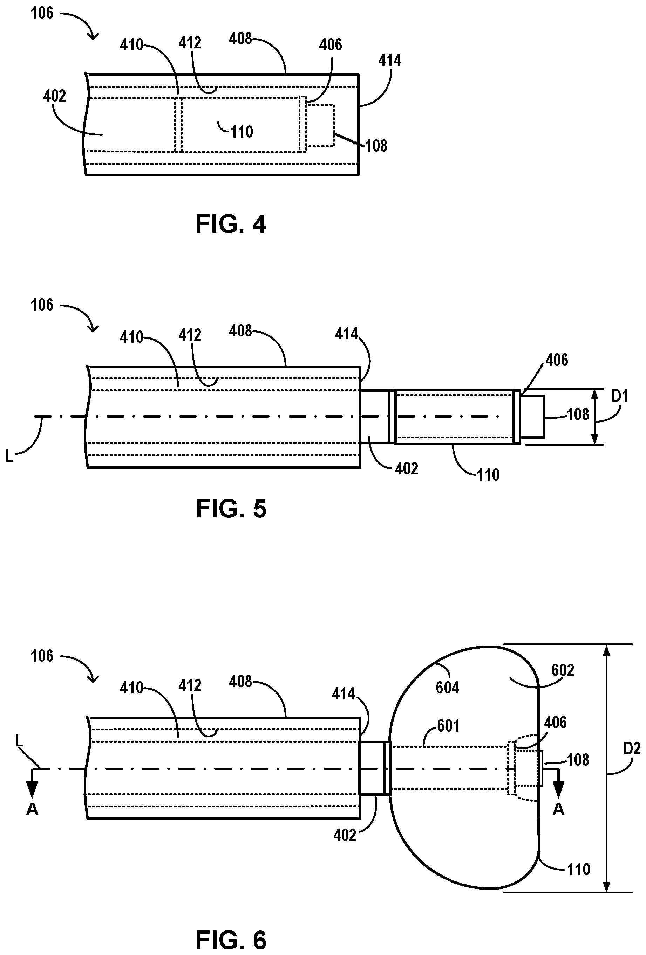

FIG. 4 is a conceptual diagram illustrating a distal portion 106 of the implantable medical lead 104 in a retracted configuration. FIG. 4 illustrates lead body 402 of implantable medical lead 104 positioned within a sheath lumen 410 of a sheath 408. Sheath 408 may protect implantable medical lead 104 from external damage and/or facilitate navigation of implantable medical lead 104 through vasculature of patient 102. In some examples, sheath 408 may be a delivery catheter. Sheath 408 includes inner wall 412 defining sheath lumen 410 and further includes a sheath opening 414 to sheath lumen 410. FIG. 4 illustrates implantable medical lead 104 with expandable member 110 in a deflated configuration. Implantable medical lead 104 is configured to translate through sheath lumen 410 when expandable member 110 is in the deflated configuration. Lead electrode 108 may be disposed on a distal end 406 of lead body 402.

In some examples, implantable medical lead 104 may be disposed within vasculature of patient 102 without use of sheath 408. In some examples, lead electrode 108 of implantable medical lead 104 may be configured to transmit electrical signals (e.g., electrical signal 302) while expandable member 110 is in the deflated configuration and/or while implantable medical lead 104 is disposed within sheath lumen 410. In some examples, sheath 408 may be configured to block or at least directionally impede the transmitted electrical signals.

FIG. 5 is a conceptual diagram illustrating distal portion 106 of implantable medical lead 104 in an extended configuration. In the example illustrated in FIG. 5, expandable member 110 is in the deflated configuration defining a maximum initial diameter D1 perpendicular to a longitudinal axis L defined by lead body 402. Medical device system 100 may define maximum initial dimension D1 to allow implantable medical lead 104 to translate through sheath lumen 410 and vasculature of patient 102 to arrive at a target area within vasculature of patient 102. As illustrated in FIG. 5, expandable member 110 may be disposed around an elongated body 502 of distal portion 106. Elongated body 502 may connect lead electrode 108 to the rest of lead body 402 of implantable medical lead 104.

FIG. 6 is a conceptual diagram illustrating distal portion 106 of implantable medical lead 104 of FIG. 5 with expandable member 110 in an expanded configuration. Expandable member 110 defines an interior volume 602 configured to contain an inflating medium (e.g., air saline, or another inflating medium) to cause expandable member 110 to transition from the deflated configuration of FIGS. 2 and 3 to the expanded configuration depicted in FIG. 4. In examples, interior volume 602 is bound at least in part by an inner surface 604 of expandable member 110 and an external surface 601 of a portion of lead body 402 of distal portion 106. Lead body 402 may define one or more inflation lumen configured to provide the inflating medium to interior volume 602.

In the expanded configuration, expandable member 110 defines a maximum expanded diameter D2 perpendicular to longitudinal axis L. The maximum expanded diameter D2 of the expanded configuration is greater than the maximum initial diameter D1 of the deflated configuration. In the expanded configuration, expandable member 110 extends distal to distal end 406 of lead body 402, with a portion of lead electrode 108 extending distal to expandable member 110. Expandable member 110 may substantially surround longitudinal axis L in the inflated configuration, such that expandable member 110 substantially forms a annular shape circumferentially around lead electrode 108. In some examples, expandable member 110 may define a substantially toroidal shape surrounding distal portion 106, distal end 406, and lead electrode 108 when expandable member 110 is in the inflated configuration. Expandable member 110 may be configured such that lead electrode 108 extends at least partially within a hole defined by the substantial toroidal shape.

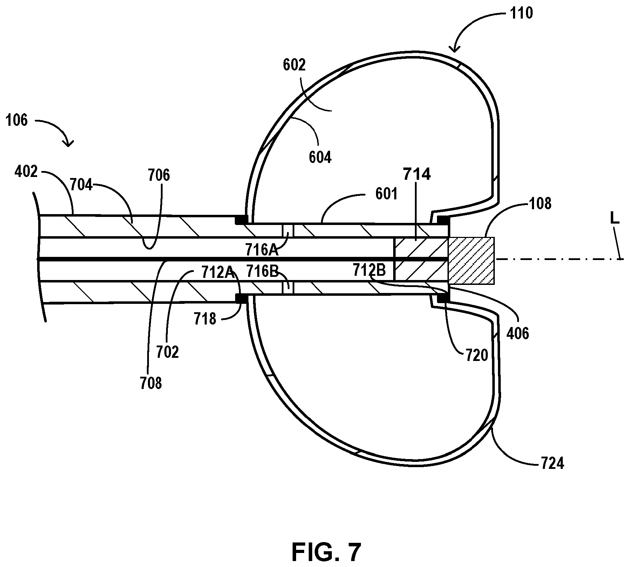

FIG. 7 is a conceptual diagram illustrating a cross-sectional view of distal portion 106 of implantable medical lead 104 of FIG. 6 along line A-A. FIG. 7 illustrated medical device system 100 with implantable medical lead 104 with expandable member 110 in the expanded configuration and having lead electrode transmitting an electrical signal. In some examples, a medical professional may navigate implantable medical lead 104 within vasculature of patient 102 with expandable member 110 in the expanded configuration. Once distal portion 106 of implantable medical lead 104 arrives at the target area within patient 102, the medical professional may then deflate expandable member 110.

Lead body 402 may define an inner lumen 702 extending at least partially through lead body 402. In some examples, longitudinal axis L extends through inner lumen 702 and intersects distal end 406. Lead body 402 may include a wall 704 defining an inner surface 706, with inner surface 706 defining inner lumen 702. Inner lumen 702 may extend to a proximal opening defined in a proximal portion of lead body 402 of implantable medical lead 104. Implantable medical lead 104 may be configured such that the proximal opening is extracorporeal to patient 102 when distal portion 106 is positioned within vasculature of patient 102.

Implantable medical lead 104 may include a conductor 708 in electrical communication with lead electrode 108. In some examples, lead electrode 108 may be in electrical communication with computing system 112. Conductor 708 may extend through inner lumen 702, although this is not required. Conductor 708 may be configured to electrically communicate with signal generation circuitry 202 (as shown in FIG. 2) to transmit electrical signals to surface electrodes 114 from lead electrode 108. In some examples, conductor 708 may be configured to extend to a position extracorporeal to patient 102 when lead body 402 extends through vasculature of patient 102, and/or when distal portion 106 is positioned at a target are of patient 102.

In some examples, distal portion 106 may be configured such that inner lumen 702 is fluidically isolated from vasculature of patient 102 when lead body 402 extends through vasculature of patient 102. Distal portion 106 may be configured such that inner lumen 702 is fluidically isolated from distal end 406 when distal end 406 is within vasculature of patient 102. In some examples, distal portion 106 may include a stop 714 configured to fluidically isolate inner lumen 702 and distal end 406 when distal end 406 is within vasculature of patient 102. In some examples, stop 714 may support lead electrode 108.

Expandable member 110 may include an expandable body 724 configured to elastically expand when expandable member 110 transitions from the deflated configuration to the expanded configuration (e.g., when an inflating medium is provided to interior volume 602). Expandable body 724 may define inner surface 604. In some examples, expandable body 724 extends at least partially around a parameter defined by external surface 601 of lead body 402 (e.g., a perimeter perpendicular to longitudinal axis L). In some examples, expandable body 724 extends substantially completely around the perimeter defined by external surface 601. Expandable body 724 (e.g., inner surface 604) and a portion of external surface 601 may define inner volume 602. Expandable body 724 and external surface 601 may define at least a portion of or in some cases substantially all of an inner boundary of inner surface 604.

Distal portion 106 may include one or more structures defining a lumen configured to deliver an inflating medium (e.g., air, saline, or another inflating medium) to interior volume 602. In some examples, lead body 402 may define lumen(s) configured to deliver the inflating medium. In some examples, inner lumen 702 may be configured to deliver the inflating medium. Distal portion 106 may include one or more side lumens such as side lumens 716A-716B (hereinafter referred to as "side lumens 716"). Side lumens 716 may be configured to fluidically couple inner lumen 702 and interior volume 602, such that the inflating medium provided to inner lumen 702 may flow to interior volume 602 via side lumens 716.

As discussed, expandable member 110 is configured to expand radially outward from external surface 601 and extend distal beyond distal end 406 when expandable member 110 expands from the deflated configuration (e.g., as shown in FIG. 5) to the expanded configuration (e.g., as shown in FIGS. 6 and 7). In some examples, expandable body 724 may be affixed to external surface 601 at least at locations 712A-712B (hereinafter referred to as "locations 712"). In some examples, expandable body 724 may be affixed to external surface 601 at a first location 712A and a second location 712B, with first location 712A proximal to second location 712B.

Expandable member 110 may be configured to cause a distal portion of expandable member 110 to extend distally beyond distal end 406 by altering an interior surface area of inner surface 604. In some examples, the inflating medium may exert a greater force against a first portion of expandable member 110 than on a second portion of expandable member 110. In some examples, the greater force may cause the first portion of expandable member 110 to "roll" distally and extend over distal end 406 and a portion of lead electrode 108 as expandable member 110 is expanded.

Distal portion 106 may include one or more fixation structures configured to affix expandable body 724 to lead body 402 (e.g., to external surface 601). In some examples, distal portion 106 may include a first fixation structure 718 configured to affix expandable body 724 at first location 712A. In some examples distal portion 724 may include a second fixation structure 720 configured to affix expandable body 724 at second location 712B. As used here, when a portion of expandable body 724 is affixed to a portion of lead body 402, this may mean distal portion 106 is configured such that the portion of expandable body 724 remains substantially stationary relative to the portion of lead body 402 as expandable member 110 transitions between the deflated state and the expanded state. In some examples, medical device system 100 may be configured to affix the portion of expandable body 724 to lead body 402 using an adhesive, soldering, welding, heat shrinking, or some other joining method.

Fixation structures 718 and 720 may define any shape sufficient to affix expandable body 724 to lead body 402, and may affix expandable body 724 to lead body 402 in any manner. In some examples, fixation structures 718 and 720 may be configured to pin a portion of expandable body 724 between lead body 402 and each respective fixation structure. In some examples, a portion of expandable body 724 may extend around a periphery of lead body 402 and first fixation structure 718 and/or second fixation structure 720 is configured to pin the portion of expandable body 724 around the periphery. In some examples, first fixation structure 718 and/or second fixation structure 720 may define a ring structure configured to surround a portion of lead body 402 and longitudinal axis L.

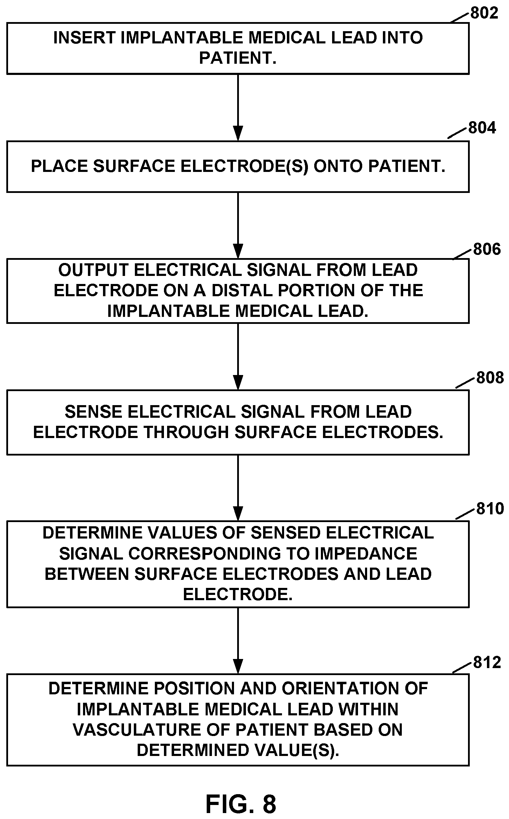

FIG. 8 is a flowchart illustrating an example process of navigating implantable medical lead 104 within body of patient 102. A user (e.g., a medical professional) may insert implantable medical lead 104 into patient 102 (802). The user may insert implantable medical lead 104 into vasculature of patient 102 at insertion site 105 on patient 102. In some examples, insertion site 105 may be on upper thorax or clavicular area of patient 102, e.g., as illustrated in FIG. 1. In other examples, insertion site 105 may be located on other portions of body of patient 102, e.g., in the groin, in the inner thigh, in the abdomen, in the neck, or the like. The user may electrically connect implantable medical lead 104 to computing system 112.

The user may place surface electrodes 114 onto patient 102 (804). In some examples, as illustrated in FIG. 1, the user may place four surface electrodes 114 onto skin of patient 102, e.g., at pectoral and pelvic regions of patient 102. In other examples, the user may place three surface electrodes 114 or five or more surface electrodes 114. In some examples, the user may place surface electrodes at other locations on patient 102, e.g., on the upper and/or lower back of patient 102.

The user may output an electrical signal (e.g., electrical signal 302) from lead electrode 108 on distal portion 106 of implantable medical lead 104 (806). In some examples, the user may instruct computing system 112 to output electrical signal 302 from lead electrode 108. The user may transmit instructions to computing system 112 via an external computing device and/or a user interface generated by computing system 112. Upon receiving instructions from the user, processing circuitry 204 of computing system 112 may retrieve instructions from memory 212 and instruct signal generation circuitry 202 of computing system 112 to transmit electrical signal 302. Signal generation circuitry 202 may output electrical signal to lead electrode 108 through conductors (e.g., conductor 708) of implantable medical lead 104. Lead electrode 108 may then output electrical signal 302 into tissue of patient 102. In some examples, lead electrode 108 may output a constant electrical signal. In some examples lead electrode 108 may output a plurality of electrical signal bursts and/or may output electrical signal 302 in response to computing system 112 receiving an instruction to do so from the user.

In some examples, the user may expand expandable member 110 of implantable medical lead 104 to an expanded configuration prior to instructing computing system 112 to transmit electrical signal 302. When expanded, portions of expandable member 110 may wrap around portion of lead electrode 108 and may block or directionally impeded portions of lead electrode 108 from transmitting electrical signal 302.

Computing system 112 may sense electrical signal 302 from transmitted from lead electrode 108 through surface electrodes 112 (808). Sensing circuitry 206 may receive the sensed electrical signals from surface electrodes 114. In some examples, one or more of surface electrodes 114 may not sense electrical signal 302 or may sense a diminished electrical signal 302 due to expandable member 110 blocking or impeding portions of lead electrode 108 from transmitting electrical signal 302. In

Computing system 112 may determine values of the sensed electrical signals corresponding to impedance between surface electrodes and lead electrode (810). In some examples, the values may include the voltage or amplitude sensed by each of surface electrodes 114.

Computing system 112 may determine the position and orientation of implantable medical lead 104 within vasculature of patient based on the determined values (812). In some examples, computing system 112 may determine a linear distance between lead electrode and each of surface electrodes 114. Computing system 112 may then triangulate the position of lead electrode 108. In some examples, computing system 112 may determine the orientation of implantable medical lead 104 based on which of surface electrodes 114 is blocked or impeded from sensing electrical signal 302 by expandable member 110 around a portion of lead electrode 108.

The techniques of this disclosure may be implemented in a wide variety of computing devices, medical devices, or any combination thereof. Any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The disclosure contemplates computer-readable storage media comprising instructions to cause a processor to perform any of the functions and techniques described herein. The computer-readable storage media may take the example form of any volatile, non-volatile, magnetic, optical, or electrical media, such as a RAM, ROM, NVRAM, EEPROM, or flash memory that is tangible. The computer-readable storage media may be referred to as non-transitory. A server, client computing device, or any other computing device may also contain a more portable removable memory type to enable easy data transfer or offline data analysis.

The techniques described in this disclosure, including those attributed to various modules and various constituent components, may be implemented, at least in part, in hardware, software, firmware or any combination thereof. For example, various aspects of the techniques may be implemented within one or more processors, including one or more microprocessors, DSPs, ASICs, FPGAs, or any other equivalent integrated, discrete logic circuitry, or other processing circuitry, as well as any combinations of such components, remote servers, remote client devices, or other devices. The term "processor" or "processing circuitry" may generally refer to any of the foregoing logic circuitry, alone or in combination with other logic circuitry, or any other equivalent circuitry.

Such hardware, software, firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. For example, any module described herein may include electrical circuitry configured to perform the features attributed to that particular module, such as fixed function processing circuitry, programmable processing circuitry, or combinations thereof.

The techniques described in this disclosure may also be embodied or encoded in an article of manufacture including a computer-readable storage medium encoded with instructions. Instructions embedded or encoded in an article of manufacture including a computer-readable storage medium encoded, may cause one or more programmable processors, or other processors, to implement one or more of the techniques described herein, such as when instructions included or encoded in the computer-readable storage medium are executed by the one or more processors. Example computer-readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or any other computer readable storage devices or tangible computer readable media. The computer-readable storage medium may also be referred to as storage devices.

In some examples, a computer-readable storage medium comprises non-transitory medium. The term "non-transitory" may indicate that the storage medium is not embodied in a carrier wave or a propagated signal. In certain examples, a non-transitory storage medium may store data that can, over time, change (e.g., in RAM or cache).

It should be noted that medical device system 100, and the techniques described herein, may not be limited to use in a human patient. In alternative examples, medical device system 100 may be implemented in non-human patients, e.g., primates, canines, equines, pigs, and felines. These other animals may undergo clinical or research therapies that my benefit from the subject matter of this disclosure.

The invention is defined by the appended claims.

## Claims

1. A computer readable storage medium comprising instructions that, when executed, cause processing circuitry within a device to perform:
outputting, by a computing system through a lead electrode on a distal portion of an implantable medical lead within vasculature of a patient, an electrical signal, wherein the implantable medical lead further comprises an expandable member that is in an expanded state while the electrical signal is outputted, wherein the expandable member is configured, when expanded, to directionally-impede the electrical signal;
sensing, by the computing system and through each of a plurality of surface electrodes positioned on the patient, the electrical signal;
determining, by the computing system for each respective surface electrode of the plurality of surface electrodes, a value of the sensed electrical signal corresponding to an impedance between the respective surface electrode and the lead electrode; and
determining, by the computing system, a position and an orientation of the distal portion of the implantable medical lead within the vasculature of the patient based on the determined values of the sensed electrical signal.

2. The computer readable storage medium of claim 1, wherein determining the position and the orientation of the distal portion of the implantable medical lead comprises:
determining, based at least in part on the determined values of the sensed electrical signal and the positions of the plurality of surface electrodes, a position of the lead electrode within the vasculature of the patient.

3. The computer readable storage medium of claim 2, wherein determining the position of the lead electrode comprises:
determining, by the computing system, differences between the determined values of the sensed electrical signal for the plurality of surface electrodes; and
triangulating, by the computing system, the position of the lead electrode based at least in part on the differences between the values and the position of each of the plurality of surface electrodes on the patient,
particularly, wherein triangulating the position of the lead electrode comprises:
determining, by the computing system, a linear distance between the lead electrode and each respective surface electrode of the plurality of surface electrodes based on the respective value of the sensed electrical signal determined for the respective surface electrode;
determining, by the computing system and for each respective surface electrode, a sphere around the surface electrode, the sphere having a diameter of the corresponding linear distance; and
determining, by the computing system, a point of intersection between the spheres of the plurality of surface electrodes, wherein the position of the lead electrode corresponds to the point of intersection.

4. The computer readable storage medium of any of claims 1-3, wherein determining the position and the orientation of the distal portion of the implantable medical lead further comprises determining the orientation of the lead electrode, and wherein determining the orientation of the lead electrode comprises:
determining, by the computing system and for each respective surface electrode of the plurality of surface electrodes, an expected sensed electrical signal value for the respective surface electrode based on the determined position of the lead electrode;
comparing, by the computing system and for each respective surface electrode of the plurality of surface electrodes, the expected sensed electrical signal value against the determined value of the sensed electrical signal;
selecting, by the computing system, one or more surface electrodes of the plurality of surface electrodes with the determined values of the sensed electrical signal substantially greater than the expected sensed electrical signal values; and
determining, by the computing system and based at least in part on the position of the lead electrode and on the positions of the one or more selected surface electrodes, the orientation of the lead electrode.

5. The computer readable storage medium of any of claims 1-4, further comprising instructions that, when executed, cause processing circuitry within a device to perform:
detecting, by the computing system, a pressure within the expandable member on the implantable medical lead;
determining, by the computing system, a direction of obscuration of the lead electrode by the expandable member based on the detected pressure; and
determining, by the computing system, the position and the orientation of the distal portion of the implantable medical lead based on the direction of obscuration.

6. The computer readable storage medium of any of claims 1-5, wherein the expandable member is a balloon.

7. The computer readable storage medium of any of claims 1-6, wherein the electrical signal comprises a plurality of electrical signal pulses or a constant electrical signal.

8. The computer readable storage medium of any of claims 1-7, further comprising instructions that, when executed, cause processing circuitry within a device to perform generating, by the computing system, a graphical display of the position and the orientation of the distal portion of the implantable medical lead on a display device.

9. A computing system comprising:
a memory;
sensing circuitry coupled to a plurality of surface electrodes placed onto skin of the patient;
signal generation circuitry coupled to a lead electrode on a distal portion of an implantable medical lead within vasculature of a patient; and
processing circuitry coupled to the memory, the sensing circuitry, and the signal generation circuitry, the processing circuitry configured to:
output an electrical signal from the lead electrode within vasculature of a patient, wherein the electrical signal is directionally impeded by an expandable member of the implantable medical lead in an expanded configuration;
sense the electrical signal through each of the plurality of surface electrodes;
determine, for each respective surface electrode of the plurality of surface electrodes, a value of the sensed electrical signal corresponding to an impedance between the respective surface electrode and the lead electrode; and
determine a position and an orientation of the distal portion of the implantable medical lead within the vasculature of the patient based on the determined values of the sensed electrical signal.

10. The computing system of claim 9, further comprising a user interface configured to display the position and the orientation of the distal portion of the implantable medical lead.

11. The computing system of any of claims 9 and 10, wherein to determine the position and the orientation of the distal portion of the implantable medical lead, the processing circuitry is configured to determine, based at least in part on the determined values of the sensed electrical signal and the positions of the plurality of surface electrodes, a position of the lead electrode within the vasculature of the patient.

12. The computing system of claim 11, wherein to determine the position of the lead electrode, the processing circuitry is further configured to:
determine differences between the determined values of the sensed electrical signal for the plurality of surface electrodes; and
triangulate the position of the lead electrode based at least in part on the differences between the values and the position of each of the plurality of surface electrodes on the patient,
particularly wherein to triangulate the position of the lead electrode, the processing circuitry is further configured to:
determine a linear distance between the lead electrode and each respective surface electrode of the plurality of surface electrodes based on the respective value of the sensed electrical signal determined for the respective surface electrode;
determine, for each respective surface electrode, a sphere around the surface electrode, the sphere having a diameter of the corresponding linear distance; and
determine a point of intersection between the spheres of the plurality of surface electrodes, wherein the position of the lead electrode corresponds to the point of intersection.

13. The computing system of any of claims 9-12, wherein to determine the position and the orientation of the distal portion of the catheter, the processing circuitry is further configured to determine the orientation of the lead electrode, wherein to determine the orientation of the lead electrode, the processing circuitry is further configured to:
determine, for each respective surface electrode of the plurality of surface electrodes, an expected sensed electrical signal value for the respective surface electrode based on the determined position of the lead electrode;
compare, for each respective surface electrode of the plurality of surface electrodes, the expected sensed electrical signal value against the determined value of the sensed electrical signal;
select one or more surface electrodes of the plurality of surface electrodes with the determined values of the sensed electrical signal substantially greater than the expected sensed electrical signal values; and
determine, based at least in part on the position of the lead electrode and on the positions of the one or more selected surface electrodes, the orientation of the lead electrode.

14. The computing system of any of claims 9-13, wherein the processing circuitry is further configured to:
detect a pressure within the expandable member on the implantable medical lead;
determine a direction of obscuration of the lead electrode by the expandable member based on the detected pressure; and
determine the position and the orientation of the distal portion of the implantable medical lead based on the direction of obscuration.

15. The computing system of any of claims 9-14, wherein the electrical signal comprises a plurality of electrical signal pulses, and/or wherein the electrical signal comprises a constant electrical signal.

## Patentansprüche

1. Computerlesbares Speicherungsmedium, umfassend Anweisungen, die, wenn sie ausgeführt werden, eine Verarbeitungsschaltung innerhalb einer Vorrichtung veranlassen, durchzuführen:
Ausgeben, durch ein Rechensystem über eine Lead-Elektrode an einem distalen Abschnitt einer implantierbaren medizinischen Lead innerhalb der Vaskulatur eines Patienten, eines elektrischen Signals, wobei die implantierbare medizinische Lead ferner ein expandierbares Element umfasst, das sich in einem expandierten Zustand befindet, während das elektrische Signal ausgegeben wird, wobei das expandierbare Element konfiguriert ist, um, wenn ausgedehnt, das elektrische Signal richtungsabhängig zu behindern;
Erfassen, durch das Rechensystem und über jede einer Vielzahl von Oberflächenelektroden, die auf dem Patienten positioniert sind, des elektrischen Signals;
Bestimmen, durch das Rechensystem für jede jeweilige Oberflächenelektrode der Vielzahl von Oberflächenelektroden, eines Werts des erfassten elektrischen Signals, das einer Impedanz zwischen der jeweiligen Oberflächenelektrode und der Lead-Elektrode entspricht; und
Bestimmen, durch das Rechensystem, einer Position und einer Orientierung des distalen Abschnitts der implantierbaren medizinischen Lead innerhalb der Vaskulatur des Patienten basierend auf den bestimmten Werten des erfassten elektrischen Signals.

2. Computerlesbares Speicherungsmedium nach Anspruch 1, wobei das Bestimmen der Position und der Orientierung des distalen Abschnitts der implantierbaren medizinischen Lead umfasst:
Bestimmen, basierend mindestens teilweise auf den bestimmten Werten des erfassten elektrischen Signals und den Positionen der Vielzahl von Oberflächenelektroden, einer Position der Lead-Elektrode innerhalb der Vaskulatur des Patienten.

3. Computerlesbares Speicherungsmedium nach Anspruch 2, wobei das Bestimmen der Position der Lead-Elektrode umfasst:
Bestimmen, durch das Rechensystem, von Unterschieden zwischen den bestimmten Werten des erfassten elektrischen Signals für die Vielzahl von Oberflächenelektroden; und
Triangulieren, durch das Rechensystem, der Position der Lead-Elektrode basierend mindestens teilweise auf den Unterschieden zwischen den Werten und der Position jeder der Vielzahl von Oberflächenelektroden an dem Patienten,
insbesondere, wobei das Triangulieren der Position der Lead-Elektrode umfasst:
Bestimmen, durch das Rechensystem, eines linearen Abstands zwischen der Lead-Elektrode und jeder jeweiligen Oberflächenelektrode der Vielzahl von Oberflächenelektroden basierend auf dem jeweiligen Wert des erfassten elektrischen Signals, der für die jeweilige Oberflächenelektrode bestimmt wird;
Bestimmen, durch das Rechensystem und für jede jeweilige Oberflächenelektrode, einer Kugel um die Oberflächenelektrode herum, wobei die Kugel einen Durchmesser des entsprechenden linearen Abstands aufweist; und
Bestimmen, durch das Rechensystem, eines Schnittpunkts zwischen den Kugeln der Vielzahl von Oberflächenelektroden, wobei die Position der Lead-Elektrode dem Schnittpunkt entspricht.

4. Computerlesbares Speicherungsmedium nach einem der Ansprüche 1 bis 3, wobei das Bestimmen der Position und der Orientierung des distalen Abschnitts der implantierbaren medizinischen Lead ferner das Bestimmen der Orientierung der Lead-Elektrode umfasst, und wobei das Bestimmen der Orientierung der Lead-Elektrode umfasst:
Bestimmen, durch das Rechensystem und für jede jeweilige Oberflächenelektrode der Vielzahl von Oberflächenelektroden, eines erwarteten erfassten elektrischen Signalwerts für die jeweilige Oberflächenelektrode basierend auf der bestimmten Position der Lead-Elektrode;
Vergleichen, durch das Rechensystem und für jede jeweilige Oberflächenelektrode der Vielzahl von Oberflächenelektroden, des erwarteten erfassten elektrischen Signalwerts gegen den bestimmten Wert des erfassten elektrischen Signals;
Auswählen, durch das Rechensystem, einer oder mehrerer Oberflächenelektroden der Vielzahl von Oberflächenelektroden mit den bestimmten Werten des erfassten elektrischen Signals, die im Wesentlichen größer als die erwarteten erfassten elektrischen Signalwerte sind; und
Bestimmen, durch das Rechensystem und basierend mindestens teilweise auf der Position der Lead-Elektrode und auf den Positionen der einen oder der mehreren ausgewählten Oberflächenelektroden, der Orientierung der Lead-Elektrode.

5. Computerlesbares Speicherungsmedium nach einem der Ansprüche 1 bis 4, ferner umfassend Anweisungen, die, wenn sie ausgeführt werden, eine Verarbeitungsschaltung innerhalb einer Vorrichtung veranlassen, durchzuführen:
Erkennen, durch das Rechensystem, eines Drucks innerhalb des expandierbaren Elements an der implantierbaren medizinischen Lead;
Bestimmen, durch das Rechensystem, einer Richtung einer Verdunkelung der Lead-Elektrode durch das expandierbare Element basierend auf dem erkannten Druck; und
Bestimmen, durch das Rechensystem, der Position und der Orientierung des distalen Abschnitts der implantierbaren medizinischen Lead basierend auf der Richtung der Verdunkelung.

6. Computerlesbares Speicherungsmedium nach einem der Ansprüche 1 bis 5, wobei das expandierbare Element ein Ballon ist.

7. Computerlesbares Speicherungsmedium nach einem der Ansprüche 1 bis 6, wobei das elektrische Signal eine Vielzahl von elektrischen Signalimpulsen oder ein konstantes elektrisches Signal umfasst.

8. Computerlesbares Speicherungsmedium nach einem der Ansprüche 1 bis 7, ferner umfassend Anweisungen, die, wenn sie ausgeführt werden, eine Verarbeitungsschaltung innerhalb einer Vorrichtung veranlassen, ein Erzeugen, durch das Rechensystem, einer grafischen Anzeige der Position und der Orientierung des distalen Abschnitts der implantierbaren medizinischen Lead auf einer Anzeigevorrichtung durchzuführen.

9. Rechensystem, umfassend:
einen Speicher;
Erfassungsschaltung, die an eine Vielzahl von Oberflächenelektroden gekoppelt ist, die auf die Haut des Patienten platziert ist;
Signalerzeugungsschaltung, die an eine Lead-Elektrode an einem distalen Abschnitt einer implantierbaren medizinischen Lead innerhalb der Vaskulatur eines Patienten gekoppelt ist; und
Verarbeitungsschaltung, die mit dem Speicher, der Erfassungsschaltlogik und der Signalerzeugungsschaltung gekoppelt ist, wobei die Verarbeitungsschaltung konfiguriert ist zum:
Ausgeben eines elektrischen Signals von der Lead-Elektrode innerhalb der Vaskulatur eines Patienten, wobei das elektrische Signal durch ein expandierbares Element der implantierbaren medizinischen Lead in einer expandierten Konfiguration richtungsabhängig behindert wird;
Erfassen des elektrischen Signals über jede der Vielzahl von Oberflächenelektroden;
Bestimmen, für jede jeweilige Oberflächenelektrode der Vielzahl von Oberflächenelektroden, eines Werts des erfassten elektrischen Signals gemäß einer Impedanz zwischen der jeweiligen Oberflächenelektrode und der Lead-Elektrode; und
Bestimmen einer Position und einer Orientierung des distalen Abschnitts der implantierbaren medizinischen Lead innerhalb der Vaskulatur des Patienten basierend auf den bestimmten Werten des erfassten elektrischen Signals.

10. Rechensystem nach Anspruch 9, ferner umfassend eine Benutzerschnittstelle, die konfiguriert ist, um die Position und die Orientierung des distalen Abschnitts der implantierbaren medizinischen Lead anzuzeigen.

11. Rechensystem nach einem der Ansprüche 9 und 10, wobei, um die Position und die Orientierung des distalen Abschnitts der implantierbaren medizinischen Lead zu bestimmen, die Verarbeitungsschaltung konfiguriert ist, um basierend mindestens teilweise auf den bestimmten Werten des erfassten elektrischen Signals und den Positionen der Vielzahl von Oberflächenelektroden eine Position der Lead-Elektrode innerhalb der Vaskulatur des Patienten zu bestimmen.

12. Rechensystem nach Anspruch 11, wobei, um die Position der Lead-Elektrode zu bestimmen, die Verarbeitungsschaltung ferner konfiguriert ist zum:
Bestimmen von Unterschieden zwischen den bestimmten Werten des erfassten elektrischen Signals für die Vielzahl von Oberflächenelektroden; und
Triangulieren der Position der Lead-Elektrode basierend mindestens teilweise auf den Unterschieden zwischen den Werten und der Position jeder der Vielzahl von Oberflächenelektroden an dem Patienten,
insbesondere wobei, um die Position der Lead-Elektrode zu triangulieren, die Verarbeitungsschaltung ferner konfiguriert ist zum:
Bestimmen eines linearen Abstands zwischen der Lead-Elektrode und jeder jeweiligen Oberflächenelektrode der Vielzahl von Oberflächenelektroden basierend auf dem jeweiligen Wert des erfassten elektrischen Signals, das für die jeweilige Oberflächenelektrode bestimmt wird;
Bestimmen, für jede jeweilige Oberflächenelektrode, einer Kugel um die Oberflächenelektrode herum, wobei die Kugel einen Durchmesser des entsprechenden linearen Abstands aufweist; und
Bestimmen eines Schnittpunkts zwischen den Kugeln der Vielzahl von Oberflächenelektroden, wobei die Position der Lead-Elektrode dem Schnittpunkt entspricht.

13. Rechensystem nach einem der Ansprüche 9 bis 12, wobei, um die Position und die Orientierung des distalen Abschnitts des Katheters zu bestimmen, die Verarbeitungsschaltung ferner konfiguriert ist, um die Orientierung der Lead-Elektrode zu bestimmen, wobei, um die Orientierung der Lead-Elektrode zu bestimmen, die Verarbeitungsschaltung ferner konfiguriert ist zum:
Bestimmen, für jede jeweilige Oberflächenelektrode der Vielzahl von Oberflächenelektroden, eines erwarteten erfassten elektrischen Signalwerts für die jeweilige Oberflächenelektrode basierend auf der bestimmten Position der Lead-Elektrode;
Vergleichen, für jede jeweilige Oberflächenelektrode der Vielzahl von Oberflächenelektroden, den erwarteten erfassten elektrischen Signalwert gegen den bestimmten Wert des erfassten elektrischen Signals;
Auswählen einer oder mehrerer Oberflächenelektroden der Vielzahl von Oberflächenelektroden mit den bestimmten Werten des erfassten elektrischen Signals, die im Wesentlichen größer als die erwarteten erfassten elektrischen Signalwerte sind; und
Bestimmen, basierend mindestens teilweise auf der Position der Lead-Elektrode und auf den Positionen der einen oder der mehreren ausgewählten Oberflächenelektroden, der Orientierung der Lead-Elektrode.

14. Rechensystem nach einem der Ansprüche 9 bis 13, wobei die Verarbeitungsschaltung ferner konfiguriert ist zum:
Erkennen eines Drucks innerhalb des expandierbaren Elements an der implantierbaren medizinischen Lead;
Bestimmen einer Richtung der Verdunkelung der Lead-Elektrode durch das expandierbare Element basierend auf dem erkannten Druck; und
Bestimmen der Position und der Orientierung des distalen Abschnitts der implantierbaren medizinischen Lead basierend auf der Richtung der Verdunkelung.

15. Rechensystem nach einem der Ansprüche 9 bis 14, wobei das elektrische Signal eine Vielzahl von elektrischen Signalimpulsen umfasst, und/oder wobei das elektrische Signal ein konstantes elektrisches Signal umfasst.

## Revendications

1. Support de stockage lisible par ordinateur comprenant des instructions qui, lorsqu'elles sont exécutées, amènent le circuit de traitement d'un dispositif à effectuer :
l'émission, par un système informatique à travers une électrode de dérivation sur une partie distale d'une sonde médicale implantable à l'intérieur du système vasculaire d'un patient, d'un signal électrique, dans lequel la sonde médicale implantable comprend en outre un élément expansible qui est dans un état expansé pendant que le signal électrique est émis, dans lequel l'élément expansible est conçu, lorsqu'il est expansé, pour entraver de manière directionnelle le signal électrique ;
la détection, par le système informatique et à travers de chacune d'une pluralité d'électrodes de surface positionnées sur le patient, du signal électrique ;
la détermination, par le système informatique pour chaque électrode de surface respective de la pluralité d'électrodes de surface, d'une valeur du signal électrique capté correspondant à une impédance entre l'électrode de surface respective et l'électrode de dérivation ; et
la détermination, par le système informatique, d'une position et d'une orientation de la partie distale de la sonde médicale implantable à l'intérieur du système vasculaire du patient en fonction des valeurs déterminées du signal électrique détecté.

2. Support de stockage lisible par ordinateur selon la revendication 1, dans lequel la détermination de la position et de l'orientation de la partie distale de la sonde médicale implantable comprend :
la détermination, en fonction au moins en partie des valeurs déterminées du signal électrique détecté et des positions de la pluralité d'électrodes de surface, d'une position de l'électrode de dérivation à l'intérieur du système vasculaire du patient.

3. Support de stockage lisible par ordinateur selon la revendication 2, dans lequel la détermination de la position de l'électrode de dérivation comprend :
la détermination, par le système informatique, de différences entre les valeurs déterminées du signal électrique détecté pour la pluralité d'électrodes de surface ; et
la triangulation, par le système informatique, de la position de l'électrode de dérivation en fonction au moins en partie des différences entre les valeurs et la position de chacune de la pluralité d'électrodes de surface sur le patient,
en particulier, dans lequel la triangulation de la position de l'électrode dérivation comprend :
la détermination, par le système informatique, d'une distance linéaire entre l'électrode de dérivation et chaque électrode de surface respective de la pluralité d'électrodes de surface en fonction de la valeur respective du signal électrique détecté déterminé pour l'électrode de surface respective ;
la détermination, par le système informatique et pour chaque électrode de surface respective, d'une sphère autour de l'électrode de surface, la sphère ayant un diamètre de la distance linéaire correspondante ; et
la détermination, par le système informatique, d'un point d'intersection entre les sphères de la pluralité d'électrodes de surface, dans lequel la position de l'électrode de dérivation correspond au point d'intersection.

4. Support de stockage lisible par ordinateur selon l'une quelconque des revendications 1-3, dans lequel la détermination de la position et de l'orientation de la partie distale de la sonde médicale implantable comprend en outre la détermination de l'orientation de l'électrode de dérivation, et dans lequel la détermination de l'orientation de l'électrode de dérivation comprend :
la détermination, par le système informatique et pour chaque électrode de surface respective de la pluralité d'électrodes de surface, d'une valeur attendue du signal électrique détecté pour l'électrode de surface respective en fonction de la position déterminée de l'électrode de dérivation ;
la comparaison, par le système informatique et pour chaque électrode de surface respective de la pluralité d'électrodes de surface, de la valeur attendue du signal électrique détecté à la valeur déterminée du signal électrique détecté ;
la sélection, par le système informatique, d'une ou plusieurs électrodes de surface de la pluralité d'électrodes de surface dont les valeurs déterminées du signal électrique détecté sont sensiblement supérieures aux valeurs attendues du signal électrique détecté ; et
la détermination, par le système informatique et en fonction au moins en partie de la position de l'électrode de dérivation et des positions de l'une ou des électrodes de surface sélectionnées, de l'orientation de l'électrode de dérivation.

5. Support de stockage lisible par ordinateur selon l'une quelconque des revendications 1-4, comprenant en outre des instructions qui, lorsqu'elles sont exécutées, amènent le circuit de traitement à l'intérieur d'un dispositif à effectuer :
la détection, par le système informatique, d'une pression à l'intérieur de l'élément expansible sur la sonde médicale implantable ;
la détermination, par le système informatique, d'une direction d'obscurcissement de l'électrode de dérivation par l'élément expansible en fonction de la pression détectée ; et
la détermination, par le système informatique, de la position et de l'orientation de la partie distale de la sonde médicale implantable en fonction de la direction d'obscurcissement.

6. Support de stockage lisible par ordinateur selon l'une quelconque des revendications 1-5, dans lequel l'élément expansible est un ballonnet.

7. Support de stockage lisible par ordinateur selon l'une quelconque des revendications 1-6, dans lequel le signal électrique comprend une pluralité d'impulsions de signal électrique ou un signal électrique constant.

8. Support de stockage lisible par ordinateur selon l'une quelconque des revendications 1-7, comprenant en outre des instructions qui, lorsqu'elles sont exécutées, amènent le circuit de traitement à l'intérieur d'un dispositif à effectuer la génération, par le système informatique, d'un affichage graphique de la position et de l'orientation de la partie distale de la sonde médicale implantable sur un dispositif d'affichage.

9. Système informatique comprenant :
une mémoire ;
un circuit de détection couplé à une pluralité d'électrodes de surface placées sur la peau du patient ;
un circuit de génération de signaux couplé à une électrode de dérivation sur une partie distale d'une sonde médicale implantable à l'intérieur du système vasculaire d'un patient ; et
un circuit de traitement couplé à la mémoire, au circuit de détection et au circuit de génération de signaux, le circuit de traitement étant configuré pour :
émettre un signal électrique à partir de l'électrode de dérivation à l'intérieur du système vasculaire d'un patient, dans lequel le signal électrique est entravé de manière directionnelle par un élément expansible de la sonde médicale implantable dans une configuration expansée ;
détecter le signal électrique à travers chacune de la pluralité d'électrodes de surface ;
déterminer, pour chaque électrode de surface respective de la pluralité d'électrodes de surface, une valeur du signal électrique détecté correspondant à une impédance entre l'électrode de surface respective et l'électrode principale ; et
déterminer une position et une orientation de la partie distale de la sonde médicale implantable à l'intérieur du système vasculaire du patient en fonction des valeurs déterminées du signal électrique détecté.

10. Système informatique selon la revendication 9, comprenant en outre une interface utilisateur configurée pour afficher la position et l'orientation de la partie distale de la sonde médicale implantable.

11. Système informatique selon l'une quelconque des revendications 9 et 10, dans lequel, pour déterminer la position et l'orientation de la partie distale de la sonde médicale implantable, le circuit de traitement est configuré pour déterminer, en fonction au moins en partie des valeurs déterminées du signal électrique détecté et des positions de la pluralité d'électrodes de surface, une position de l'électrode de dérivation à l'intérieur du système vasculaire du patient.

12. Système informatique selon la revendication 11, dans lequel, pour déterminer la position de l'électrode de dérivation, le circuit de traitement est en outre configuré pour :
déterminer des différences entre les valeurs déterminées du signal électrique détecté pour la pluralité d'électrodes de surface ; et
trianguler la position de l'électrode de dérivation en fonction au moins en partie des différences entre les valeurs et la position de chacune de la pluralité d'électrodes de surface sur le patient,
en particulier dans lequel, pour trianguler la position de l'électrode de dérivation, le circuit de traitement est en outre configuré pour :
déterminer une distance linéaire entre l'électrode de dérivation et chaque électrode de surface respective de la pluralité d'électrodes de surface en fonction de la valeur respective du signal électrique détecté déterminé pour l'électrode de surface respective ;
déterminer, pour chaque électrode de surface respective, une sphère autour de l'électrode de surface, la sphère ayant un diamètre de la distance linéaire correspondante ; et
déterminer un point d'intersection entre les sphères de la pluralité d'électrodes de surface, dans lequel la position de l'électrode de dérivation correspond au point d'intersection.

13. Système informatique selon l'une quelconque des revendications 9-12, dans lequel, pour déterminer la position et l'orientation de la partie distale du cathéter, le circuit de traitement est en outre configuré pour déterminer l'orientation de l'électrode de dérivation, dans lequel pour déterminer l'orientation de l'électrode de dérivation, le circuit de traitement est en outre configuré pour :
déterminer, pour chaque électrode de surface respective de la pluralité d'électrodes de surface, une valeur attendue du signal électrique détecté pour l'électrode de surface respective en fonction de la position déterminée de l'électrode de dérivation ;
comparer, pour chaque électrode de surface respective de la pluralité d'électrodes de surface, la valeur attendue du signal électrique détecté à la valeur déterminée du signal électrique détecté ;
sélectionner une ou plusieurs électrodes de surface de la pluralité d'électrodes de surface dont les valeurs déterminées du signal électrique détecté sont sensiblement supérieures aux valeurs attendues du signal électrique détecté ; et
déterminer, en fonction au moins en partie de la position de l'électrode de dérivation et des positions de l'une ou plusieurs électrodes de surface sélectionnées, l'orientation de l'électrode de dérivation.

14. Système informatique selon l'une quelconque des revendications 9-13, dans lequel le circuit de traitement est en outre configuré pour :
détecter une pression à l'intérieur de l'élément expansible sur la sonde médicale implantable ;
déterminer une direction d'obscurcissement de l'électrode de dérivation par l'élément expansible en fonction de la pression détectée ; et
déterminer la position et l'orientation de la partie distale de la sonde médicale implantable en fonction de la direction d'obscurcissement.

15. Système informatique selon l'une quelconque des revendications 9-14, dans lequel le signal électrique comprend une pluralité d'impulsions de signal électrique, et/ou dans lequel le signal électrique comprend un signal électrique constant.
